# EUROPEAN PATENT APPLICATION

(11) **EP 1 164 185 A2**
(43) Date of publication of application: **19.12.2001**
(21) Application number: 01305081.0
(22) Date of filing: 11.06.2001
(51) Int. Cl.: C12C 11/00, C12C 13/02, C12M 1/12

(54) **Method and apparatus for reducing foaming during fermentation**

(30) Priority: 13.06.2000 GB 0014435
(71) Applicant: AIR PRODUCTS AND CHEMICALS, INC., Allentown, PA 18195-1501 (US)
(72) Inventor: Brown, David Geoffrey, Nantwich, Cheshire (GB)
(74) Representative: Burford, Anthony Frederick

(57) **Abstract**

The concentration of gas generated in an anaerobic fermenting liquid is controlled, preferably maintained below saturation, by removal of dissolved gas by diffusion during at least part of the fermentation. The removal of gas reduces the amount of foam produced by the fermentation and provides a source of gas for downstream treatment of a fermentation product or export from the fermentation process. The invention has particular application to fermentation processes generating carbon dioxide, especially brewing beer.

## Description

The present invention relates to a process for anaerobic fermentation in which the concentration of dissolved gases, particularly carbon dioxide or methane, produced during the fermentation is controlled. The present invention is particularly relevant to the control of foaming during the brewing of beer.

Fermentation is a process in which a chemical change in a substance is induced by a microorganism, for example yeast or bacteria. The products formed depend on the fermentation conditions as well as the substance being fermented. For example, under anaerobic conditions, sugar is converted into ethanol whereas, under aerobic conditions, ethanol is converted into acetic acid.

In brewing beer, anaerobic conditions are required. Under such conditions, water and carbon dioxide are produced in addition to ethanol. Some of the carbon dioxide produced remains dissolved in the liquid part of the broth. However, when the liquid reaches saturation during fermentation, i.e. contains the maximum possible amount of dissolved carbon dioxide, the excess carbon dioxide causes foaming.

Foaming during the fermentation process is undesirable for a number of reasons. A modern fermentation vessel ("FV") in a brewery is only utilised from 65% to 75% of its capacity during fermentation because of the production of the foam. The FV is not operating at its maximum potential thereby reducing the overall efficiency of the brewing process. In addition, foaming causes components of the broth to be lost on the inside walls of the FV. For this reason, the concentration of natural chemicals involved in the stabilisation of the foam is reduced. This can necessitate the addition of foreign chemicals, for example alginates in the case of brewing beer, at a later stage in production. This results in a corresponding increase in the cost of production. In addition, the extra process steps result in the process taking longer to complete and the resultant beer can contain chemicals that would not otherwise have been present.

Various methods have been proposed to remove dissolved carbon dioxide from a fermenting liquid. It has been proposed (*J. Inst*. *Brew.,* May-June 1976, Vol. 82, pp. 168-169) that controlled release of dissolved carbon dioxide during fermentation could be induced by lowering into the liquid a unit having a specific type of surface possessing a high density of bubble nucleation sites. However, rather than preventing foaming, this method actually promotes foaming and therefore is unsuitable in addressing the present problem.

It has also been reported (*Biotechnology Letters,* 1986, Vol. 8, No. 11, pp. 811-816) that carbon dioxide may be removed from a fermenting liquid by sparging the liquid with nitrogen. The use of nitrogen for carbon dioxide stripping increases foaming, creating or intensifying the original problem since nitrogen foam is very stable. This disclosure does not, therefore, provide a solution to the foaming problem.

US-A-3992293 discloses a method of chemically stripping carbon dioxide both during fermentation and at other stages of processing. This is achieved by reaction of the dissolved carbon dioxide with soluble hydroxide to form an insoluble carbonate precipitate which is then filtered off from the liquid. Chemical stripping can be used to achieve similar results as the present invention but has additional cost from the soluble hydroxide and removal/disposal of the precipitate.

It is well known that dissolved compounds may be removed from solution using gas permeable membranes. US-A-5464540 discloses a process for the removal of at least one component of a liquid mixture involving directing the liquid mixture against the feed side of a membrane and directing a condensable vapour stream against the permeate side of the membrane such that the fluid flows are substantially countercurrent. If the components' partial pressure on the feed side of the membrane is greater than its partial pressure on the permeate side of the membrane then the component will diffuse across the membrane to the permeate side, thereby being removed from the liquid.

For the purpose of removing dissolved gases from a solution, the gas permeable membrane may take the form of a hollow fibre. Separator units comprising a number of these hollow fibres are commonly used. For example, a separator unit for in-line gas control is marketed by Realm Products Limited of Gladstone Road, Croydon, CR0 3BQ, England under the trade mark HYDROBRANE.

US-A-5254143 discloses the use of a separator unit comprising hollow fibre membranes having two surfaces. At least one surface of the membrane is hydrophilic and the surface of the pores within the membrane are hydrophobic. The membranes are suitable for dissolving a gas into a liquid. The use of a hydrophilic membrane with aqueous solutions presents inherent disadvantages, e.g. the hydrophilic surface of the membrane will dissolve in aqueous solution. This is clearly undesirable for many applications, particularly in the brewing industry.

DE-A-4143314 discloses a process for the dealcoholisation of beer by pervaporation. However, before pervaporation, the carbon dioxide is removed from the beer using a gas permeable membrane. After pervaporation, the beer is carbonated by the dissolution of carbon dioxide into the beer through a gas permeable membrane.

A process for the deaeration of liquids, including beers, using hollow fibre membrane technology is disclosed in US-A-5522917. In addition, US-A-5565149 and EP-A-0732142 both disclose processes in which carbon dioxide can be removed from beer and replaced with nitrogen using hollow fibre membranes after production of the beer. The process disclosed in US-A-5565149 is particularly suitable for the addition of a gas, usually N₂, to a beer product just before serving to ensure the beer has a good head. However, none of these publications discloses a process for the control of foaming during fermentation.

A process for the removal of carbon dioxide using a membrane during continuous aerobic fermentation of rhamnolipids by *Pseudomonas aeruginosa* is disclosed (Th. Gruber & H. Chmiel "Coupling of production and downstream processing in the continuous rhamnolipid fermentation - by Pseudomonas aeruginosa using a membrane for cell recycle and another membrane for aeration",; DECHEMA Biotechnology Conferences 4, Part B, p. 1085-1088, 1990) in which productivity was improved by using a second membrane which combined the filtration and purification steps into the FV. Two external membrane loops and a continuous FV are used. The first membrane is used to filter and recycle the microorganisms and product back to the FV. The second membrane is used to supply molecular oxygen and remove carbon dioxide from the fermentation broth. The filtration membrane is used to recycle cells thereby allowing adjustment of the cell growth rate to a low value. Lower values allow optimum product production rates while maintaining higher cell concentrations. By also using the membrane to filter and return product to the FV, the quantity and concentration of the two product streams (one from the FV and one from the permeate stream) can be controlled. The use of a second membrane allows the removal of carbon dioxide to handle higher product (a surfactant) concentrations that result in the FV and the product stream from the FV without adding antifoam agents. There is no disclosure of use of a gas permeable membrane to remove carbon dioxide from a liquid during anaerobic fermentation to reduce foaming.

The conventional approach in addressing the problem of the foaming of a fermenting liquid is to allow the foam to form and then to either remove the foam from the FV or to destroy the foam thereby allowing the portion of the fermenting liquid used to form the foam to return to the bulk of the fermenting liquid.

Removal of the foam can be achieved mechanically using a scraping device that is periodically moved over the surface of the fermenting liquid thereby removing the foam to one side of the FV. This method can result in unacceptably high levels of wastage and in the unwanted removal of natural foam stabilising chemicals from the fermenting liquid.

Once the foam has formed, it can be broken down, allowing the liquid part of the foam to rejoin the bulk of the fermenting liquid. This reduces the amount of wastage lost during processing and maintains the concentration of natural foam stabilising chemicals. Break down of the foam can be achieved chemically by adding anti-foam agents to the fermenting liquid or mechanically using, for example, a rotating blade sweeping over the surface of the fermenting liquid. The addition of anti-foam agents increases the processing costs, adds a further step to the process and can increase processing time. In addition, the fermenting liquid contains chemicals which it otherwise would not have contained and which may have to be removed after fermentation thereby adding a further step to the process. The use of a mechanical device to break down the foam increases the overall capital cost and is not particularly efficient, especially with stabilised foams.

Rather than removing the foam once it has formed, the problem of foaming has been addressed in the present invention by minimising or preventing formation of the foam during fermentation. Conventionally, foaming has been limited by the addition of anti-foam agents. However, this does not overcome all of the above-mentioned disadvantages.

It is, therefore, an object of the present invention to minimise or prevent foam from forming during a fermentation process without undue wastage, without removing the natural foam stabilisers from the fermenting liquid and without having an adverse effect on processing cost or time.

During the early stages of beer brewing fermentation, aerobic conditions can be used to provide a benefit to the fermentation performance. Oxygen addition during the early stage of fermentation is currently achieved by direct sparging into the base of the FV. However, due to the mass transfer limitations and venting losses associated with bubbles, this method only has an efficiency of around 20%.

After the early stages of beer brewing fermentation, anaerobic conditions develop. During the bulk of the fermentation process, the broth is left unagitated. This helps maintain the anaerobic conditions. However, to ensure that fermentation is complete at the end of the fermentation cycle, the broth can be agitated to get better contact between the remaining active microorganism and the sugar. Conventionally, this is achieved by bubbling a gas such as nitrogen through the broth or by using a centrifugal pump in an external recycle loop. The use of nitrogen adds additional cost to the process and helps create problematic foam. If a centrifugal pump in an extemal loop is used, the recycled portion is typically sprayed over the fermentation. This results in air being entrained into the broth that can result in aerobic fermentation thereby producing unwanted acetic acid rather than ethanol.

There are many factors that determine the productivity of a fermentation process. These factors include the amount of microorganism present and reaction temperature. Over the years, the optimum conditions for maximum productivity have been established. Therefore, it is a further object of the present invention that any improvements to the process have minimal effect on these established conditions.

The objects of the present invention have been achieved at least in part by subjecting at least a portion of the fermenting liquid to a partial pressure differential across a gas permeable membrane. Accordingly and with regard to the first aspect of the present invention, there is provided a process for anaerobic fermentation in a liquid medium wherein the concentration of gas produced by the fermenting liquid is controlled for at least part of the process by removing dissolved gas directly from the fermenting liquid by diffusion. Preferably the control maintains the concentration of dissolved gas below the saturation level.

With regard to a second aspect of the present invention, there is provided a process for reducing the level of foam generated during anaerobic fermentation in a liquid medium comprising controlling the concentration of generated gas in the fermenting liquid, preferably to below the saturation level, by removing dissolved gas therefrom by diffusion during at least part of the fermentation.

In addition to overcoming the disadvantages and drawbacks of the prior art, the present invention also provides several advantages over the prior art. In this connection, the recovered gas may either be used in downstream processing, for example, in brewing beer, for movement of product, packaging or pH adjustment, or be sold as a product (a large brewery can produce 10,000 tons of carbon dioxide per year). As the gas has not been contaminated with air, expensive purification techniques are not required.

In addition, carbon dioxide is known to inhibit the conversion of raw materials into fermentation products. Therefore, removal of the carbon dioxide removes this inhibition.

Further, the removal of one of the fermentation reaction products has the effect of driving the reaction to completion. The removal of gas generated during fermentation, therefore, increases the efficiency of the fermentation process.

In the case of brewing beer, after fermentation, carbon dioxide is used in the downstream processing of beer. Carbon dioxide is usually purchased for such use while the carbon dioxide produced during fermentation is typically vented to the atmosphere. There are cases where newly formed carbon dioxide is recovered for reuse. However, the current recovery systems just recover the off-gas from the FV headspace and therefore do not reduce or prevent foaming or the loss of foam stabilisers as these systems do not remove dissolved carbon dioxide from the fermenting liquid.

The invention minimises and/or prevents foam formation in a FV during fermentation. In addition, the present invention can drive a fermentation reaction to completion by the removal of dissolved gas to a level less than the saturation level of the fermenting liquid. Further, natural carbon dioxide or other generated gas may be recovered from a fermentation process.

The invention uses a gas permeable membrane. The membrane may have any suitable form although sheet or hollow fibre membranes are preferred. The membrane can be located either in a pumped-loop (which is external to the FV) or as a 'drop-in' unit situated inside the vessel. The latter unit could be used with or without a pump or other agitation device and in either enclosed form or with the fibres dispersed within the liquid fermentation medium.

Preferably, the fermentation liquid passes through the shell side (outside) of the membrane(s). The gas is removed from the fermentation liquid by passing the liquid over one side of the membrane(s) and by drawing the gas through the membrane to the other side. In the embodiment of the hollow fibre tube, the gas would be drawn into the lumen of the fibre(s). The gas can be drawn to the lumen by applying a pressure differential, for example using a vacuum within the lumen or pressurising the liquid on the shell side of the membrane(s) or by sweeping the lumen with another gaseous material (containing minimal amounts of the gas being extracted) to maintain a high concentration gradient of the gas being extracted across the membrane.

If the separator unit is external to the FV, the fermentation liquid exits the separation unit depleted of gas and is recycled back to the FV. The gas recovered from the tube side of the membranes can either be reused directly or processed further depending on the desired purity.

In accordance with a third aspect of the present invention, there is provided the use of a gas permeable membrane in an anaerobic fermentation process to remove dissolved gas generated in a fermenting liquid to reduce the level of foam generated by controlling the concentration of gas in the liquid, preferably to below the saturation level.

In accordance with a fourth aspect of the present invention there is provided apparatus for carrying out the process of the first or second aspect of the present invention. The apparatus comprises a fermentation vessel for anaerobic fermentation in a liquid medium, a separator unit comprising at least one gas permeable membrane for the diffusion of gas from dissolution in the fermenting liquid, means for contacting the fermenting liquid with one side of the membrane; and means for removing gas diffusing through the membrane to the other side thereof.

In one embodiment of the invention in which the separator unit is external to the fermenting vessel and connected thereto using an external loop, the apparatus further comprises:
conduit means for feeding fermenting liquid from the fermentation vessel to the separator unit; and
conduit means for removing fermenting liquid from the separator unit and returning the treated liquid to the fermentation vessel.

In an alternate embodiment, the separator unit is a "drop-in" unit located within the fermentation vessel. This embodiment has certain advantages in that the gas permeable membranes are in direct contact with the fermenting liquid inside the vessel so that the extra capital cost of conduit means from the vessel to the separator unit and from the separator unit to the vessel is avoided.

In a preferred embodiment, the separator unit further comprises means for applying a vacuum in the lumen of the hollow fibres. In this embodiment, the means for applying a vacuum comprises a vacuum pump, a closure sealing one end of the lumen of the fibres and conduit means connecting the other end of the lumen of the fibres to the vacuum pump wherein, in use, the vacuum pump draws the atmosphere from the lumen of the hollow fibres to form a vacuum.

In a further preferred embodiment, the separator unit comprises means for applying a flow of gas at a second partial pressure through the lumen of the fibres. In this embodiment, the means for applying a flow of gas comprises a reservoir of gas, gas inlet means on the separator unit leading to the inlet end of the lumen of each hollow fibre, gas outlet means on the separator unit leading from the outlet end of the lumen of each hollow fibre, conduit means for feeding gas from the reservoir to the gas inlet means and conduit means for removing gas from the gas outlet means wherein, in use, gas is fed from the reservoir and through the lumen of the hollow fibres.

The efficiency of oxygen addition in the early stages of the fermentation cycle can be improved by introducing a molecular oxygen-containing gas, usually air or, especially, oxygen, into the fermenting liquid by diffusion through the same or different separator unit used to remove dissolved gas.

The following is a description by way of example only and with reference to the accompanying drawings of presently preferred embodiments of the invention. In the drawings:
Figure 1 is a schematic representation of an apparatus for carrying out a first embodiment of the process of the present invention using a flow of nitrogen gas;
Figure 2 is a schematic representation of a part of the apparatus of Figure 1;
Figure 3 is a schematic representation of an apparatus for carrying out a second embodiment of the process of the present invention using a vacuum;
Figure 4 is a schematic representation of an alternative arrangement of the apparatus for carrying out the second embodiment of the process using a vacuum; and
Figure 5 is a graph depicting the specific gravity of beer as a function of time for six test fermentation runs.

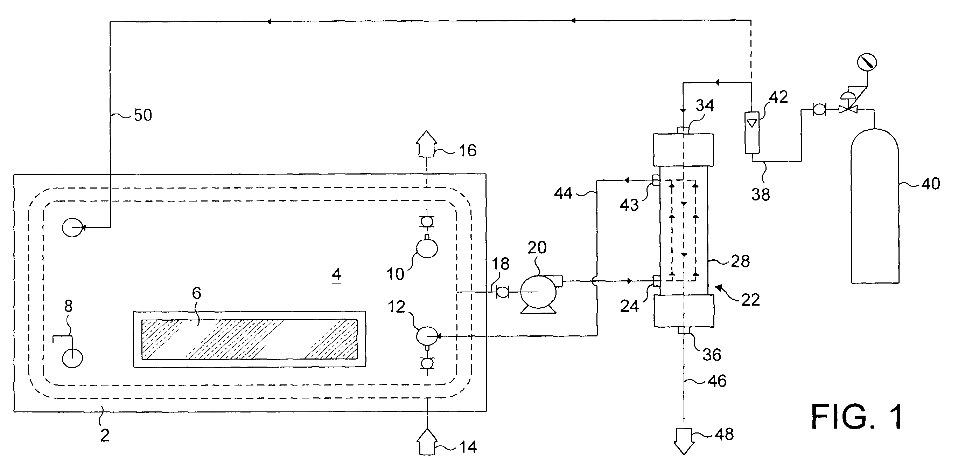

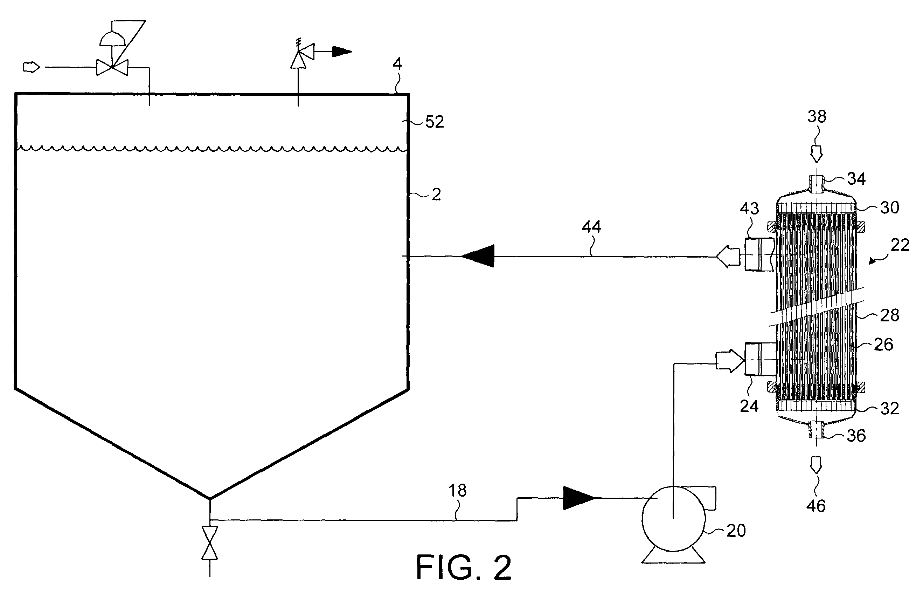

Referring to Figures 1 and 2, a beer brewing fermentation vessel 2 is provided with a lid 4, which comprises a viewing window 6. The liquid to be fermented is provided within the fermentation vessel. The vessel further comprises a water trap/vent 8 and at least two dip tube sample points 10,12.

Heat is produced as a by-product of the biological fermentation process. However, it is important that the temperature of the fermenting liquid is maintained at a constant level. Therefore, the vessel is also provided with a cooling system. In Figure 1, this is represented by a flow of coolant into 14 and out of 16 the fermentation vessel.

A stream of fermenting liquid 18 is removed from the fermentation vessel. The pressure of this stream is boosted by a pump 20 and then the stream is fed to the interior of separator unit 22 via inlet 24. The separator unit comprises a number of hollow fibre gas permeable membranes 26 contained within an outer casing 28. The fermenting liquid fills the volume inside the separator unit defined by the inner wall of the casing, and the outer walls of the hollow fibres and the inner surfaces of end walls 30 and 32. The fibres extend through the first end wall 30, through the interior of the separator unit and then through the second end wall 32. The separator unit also comprises a gas inlet 34, in communication with the inlet end of the fibre lumen, and a gas outlet 36, in communication with the outlet end of the fibre lumen.

A stream of nitrogen 38 is removed from a reservoir 40 and fed, via a valve/flow meter 42, to the inlet 34 of the separator unit 22. This nitrogen stream passes through the lumen of the fibres. The partial pressure of carbon dioxide within the nitrogen stream is lower than the partial pressure of carbon dioxide dissolved in the fermenting liquid. Accordingly, carbon dioxide permeates across the gas permeable membrane from the fermenting liquid to the lumen of the fibres. The stream of carbon dioxide-depleted liquid 44 is then removed from the separator unit via outlet 43 and fed back into the fermentation vessel. . The stream of carbon dioxide-enriched nitrogen gas 46 is removed from the separator unit via outlet 36 and may be vented or recovered 48. Nitrogen from the reservoir 40 can also be fed as a stream 50 to the fermentation vessel before or during the brewing process to maintain a positive inerting pressure within the headspace 52 of the vessel.

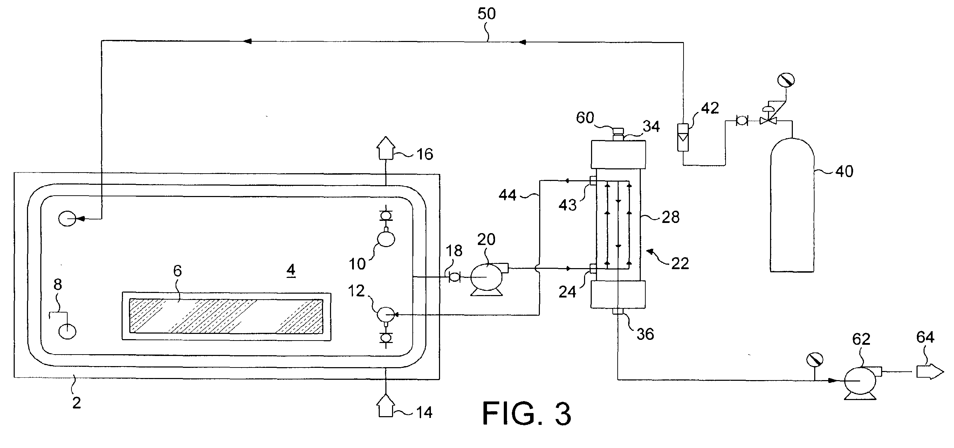

The embodiment of the present invention depicted in Figure 3 uses a vacuum to remove dissolved carbon dioxide from the fermenting liquid. The apparatus is generally the same as that for the embodiment depicted in Figure 1. However, the gas inlet 34 of the separator unit 22 is sealed 60 and the gas outlet 36 is connected to a pump 62. In use, the pump creates a vacuum within the lumen of the hollow fibres of the separator unit and the resultant pressure differential causes the carbon dioxide molecules dissolved in the fermenting liquid to permeate across the gas permeable membrane. The carbon dioxide gas extracted in this way is then recovered as a gas stream 64.

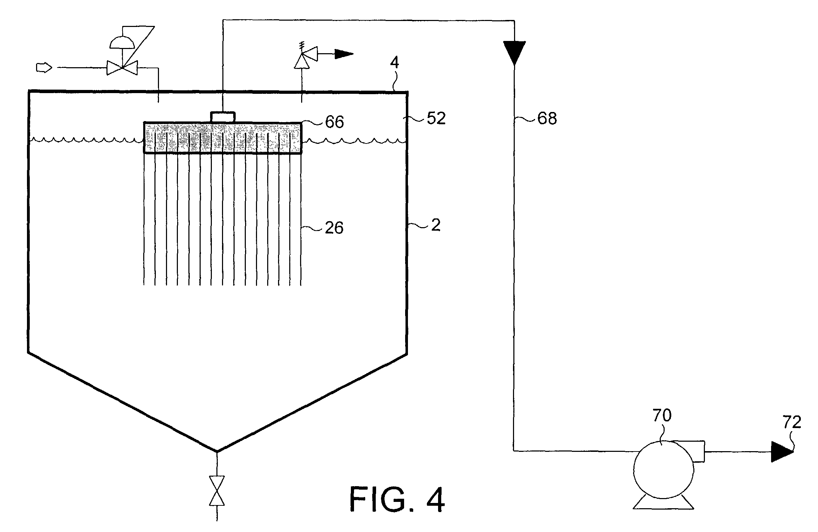

Figure 4 depicts an alternative arrangement of the apparatus used for carrying out the embodiment of the process shown in Figure 3. In this arrangement, separator unit 66 is at least partially submerged in the fermenting liquid in the FV 2. The hollow fibre membranes 26 of the separator unit are in direct contact with the main body of the fermenting liquid. The dissolved carbon dioxide may be removed from the liquid across the gas permeable membrane using either a flow of gas, for example nitrogen, or using a vacuum. In the illustrated arrangement, conduit 68 connects the lumen of the hollow fibres of the separator unit to a vacuum pump 70. Carbon dioxide is recovered as a stream 72.

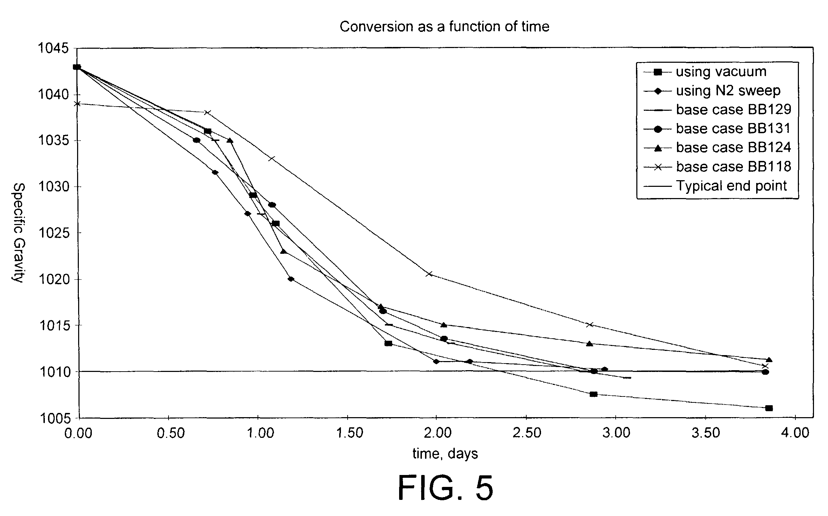

Comparative tests were carried out to demonstrate the effectiveness of the present invention. Six fermentation runs were completed and the results of these runs are shown in Figure 5. Each fermentation run used an equivalent 13 hectolitre (1300 l) brewing capacity fermentation vessel to produce a 4% alcohol by volume beer (to a standard English bitter recipe). Four of the runs were conducted using the brewery's standard brewing process but the remaining four runs were in accordance with the present invention. Two of the remaining runs were conducted using different embodiments of the invention but otherwise using the breweries standard brewing process. One run used the embodiment of the invention in which carbon dioxide is removed by applying a vacuum to the lumen of the membrane fibres with the membrane unit in the external loop arrangement and one run used to embodiment in which a stream of N₂ was fed through the fibre lumen also with the membrane unit in the extemal loop arrangement.

It is not unusual for foams to reach a height of over 17 inches (43 cm) in the test vessels. Indeed, it is known for fermentation batches to "foam over" from time to time. During the test fermentations, the depth of the fermenting liquid in the fermentation vessel was 22.5 inches (57 cm).

The results of these comparative tests indicate that the present invention has a significant effect on the level of foam production during beer fermentation. The removal of gaseous products from the fermentation, especially to a level below saturation, reduces / prevents foaming significantly.

Table 1 and 2 shows the effect on the level of foam produced during beer fermentation using the present invention. Table 1 shows the results for the embodiment of the present invention in which carbon dioxide is removed using a vacuum in the lumen of the hollow fibre membranes in the separator unit. Table 2 shows the results for the embodiment in which a flow of nitrogen through the fibre lumen is used to remove the dissolved carbon dioxide.

**TABLE 1**

| **Date / Time** | **time, days** | **foam height using vacuum** |
|---|---|---|
| 11/30/98 15:00 | 0.00 | start of brew |
| 11/30/98 17:00 | 0.08 | 5.5 in (14 cm) |
| 12/1/98 8:30 | 0.73 | No head, beer exposed |
| 12/1/98 14:30 | 0.98 | Signs of thin film / head |
| 12/1/98 17:30 | 1.10 | Signs of thin film / head |
| 12/2/98 8:30 | 1.73 | No head, signs of movement / flow from recirculation pump |
| 12/3/98 12:00 | 2.88 | * |
| 12/4/98 11:30 | 3.85 | * |

| | | |
|---|---|---|
| * - head was not recorded on batch sheets, believed continued to end with no head | | |

Using vacuum in the membrane fibre lumen results in a very low initial foam height. Soon after, the foam height is reduced to around zero as the carbon dioxide is removed for the fermentation.

**TABLE 2**

| **Date / Time** | **time, days** | **foam height using N2 sweep** |
|---|---|---|
| 10/8/98 15:00 | 0.00 | start of brew |
| 8/10/9822:00 | | * |
| 10/9/98 9:30 | 0.77 | * |
| 10/9/98 13:45 | 0.95 | More stable |
| 10/9/98 16:30 | 1.06 | * |
| 10/9/9819:30 | 1.19 | * |
| 10/10/98 15:00 | 2.00 | Head dropped 2 in (5 cm), wet and white |
| 10/10/98 19:30 | 2.19 | Jumping |
| 10/11/98 13:30 | 2.94 | Head dropped 8 in (20 cm) |

| | | |
|---|---|---|
| * - head was not recorded on batch sheets, head believed to be about 10-12 inches (25-30 cm) | | |

In the case of the nitrogen sweep in the fibre lumen, the foam level reached a height of over 10 inches (25 cm). This indicates that there is a period when the level of carbon dioxide produced during fermentation exceeds the carbon dioxide-saturation level of the fermenting liquid. When carbon dioxide production slows down, the removal using the nitrogen sweep is able to keep up with the rate of carbon dioxide production and the foam level drops. This drop in level is due to the escape of carbon dioxide to the atmosphere from the foam and/or to the dissolution of carbon dioxide back into the fermenting liquid.

The removal of one or more gaseous products drives the reaction to completion at a faster rate and to a more complete conversion. Figure 5 is a graph depicting the specific gravity during beer fermentation as a function of time for the test runs. The base cases are the four test runs which did not use the present invention to reduce the level of foam.

Specific gravity is used to monitor conversion of sugar to ethanol in the beer fermenting process. Typically, beer fermentation stops at 3-4 days and reaches a specific gravity of about 1010. The fermentation stops due to the inhibitory effects of the carbon dioxide and the condition of the biomass (i.e. age and settlement). Even if the fermentation is agitated, it still typically stops at or before a specific gravity of about 1010 due to the inhibitory effect of carbon dioxide.

With the removal of the carbon dioxide, the inhibitory effect is removed and the fermentation proceeds to a higher conversion. In three of the base cases shown in Figure 5 (produced without carbon dioxide removal), the fermentation takes 2.8 to 5 days to reach a specific gravity of 1010. For base case BB124, the specific gravity of the fermented liquid only reached 1011.25 (even with agitation) and fermentation was ended at 3.85 days. In all four of the base cases, the fermentation was agitated from late in the first day until the third or fourth day. Agitation increases fermentation rate and can aid in reaching a specific gravity of 1010. However, it can also introduce air to the fermentation broth which results in the production of acetic acid instead of ethanol.

In the case of the removal of carbon dioxide using vacuum, the conversion reached a specific gravity of 1006 and it would have proceeded further if cooling had not been applied to stop the conversion near the normal end point. In addition, the removal of the inhibitory carbon dioxide throughout the fermentation increased the rate of conversion. A conversion to a specific gravity of 1010 is reached after 2.35 days, 0.5 to 1.5 days less than the runs without carbon dioxide removal

In the case of carbon dioxide removal using nitrogen as a sweep gas in the lumen of the fibres, a conversion of 1010 is reached after 3 days compared to 3 to 4 days without carbon dioxide removal.

The difference in the fermentation rate and final conversion by the two different embodiments of the present invention is due to the fact that there is a higher carbon dioxide removal capability with the vacuum system. Therefore, in the case of the nitrogen sweep, carbon dioxide in the fermentation was above the saturation level for a long period whereas in the case of vacuum extraction, the carbon dioxide level was near or below saturation throughout the fermentation. This can be seen from the difference in foam heights shown in Table 1 and 2 above and from the gas removal data shown in Tables 3 and 4 below. With a system sized appropriately, the same results can be obtained using the nitrogen sweep as those obtained using vacuum for carbon dioxide removal and both can remove essentially all carbon dioxide if sized correctly.

**TABLE 3**

| **Gas removal using N**_{**2**} **sweep, litres/min** | | | |
|---|---|---|---|
| **time, days** | **N**_{**2**} **flow, litres/min** | **gas out, litres/min** | **Gas removal (N**_{**2**} **sweep), litres/min** |
| 0.00 | 1 | 1.0 | 0.0 |
| 0.00 | 1 | 1.0 | 0.0 |
| 0.77 | 1 | 11.0 | 10.0 |
| 0.95 | 1 | 10.0 | 9.0 |
| 1.06 | 1 | 10.0 | 9.0 |
| 1.19 | 1 | 12.0 | 11.0 |
| 2.00 | 1 | 7.5 | 6.5 |
| 2.19 | 1 | 6.0 | 5.0 |
| 2.94 | 1 | 5.0 | 4.0 |

The gas removal rates for nitrogen -sweep are equivalent to a peak of 0.8 kg/hour which corresponds well to the expected production rate based on a fermentation vessel volume of 8 barrels (1300 litres) and a typical carbon dioxide production rate for beer (for the fermentation of a 4% alcohol by volume beer, this is an average of 0.86 kg per hour over the active 48 hour period of fermentation (***ref****.*)). The gas removal rates for vacuum are high and suggest air ingress on the suction side of the vacuum pump.

**TABLE 4**

| **Gas removal using vacuum, litres/min** | | |
|---|---|---|
| **time, days** | **Vacuum in Hg (kPa)** | **Vacuum Gas removal, litres/min** |
| 0.00 | | 0.0 |
| 0.08 | 26 (88) | 3.0 |
| 0.73 | 23 (78) | 17.0 |
| 0.98 | 22 (75) | 22.0 |
| 1.10 | 20 (68) | offscale |
| 1.73 | 21 (71) | offscale |
| 2.88 | 15 (51) | offscale |
| 3.85 | 0 (0) | offscale |

It will be appreciated that the invention is not restricted to the details described above with reference to the preferred embodiments but that numerous modifications and variations can be made without departing from the scope of the invention as defined in the following claims.

## Claims

1. A process for anaerobic fermentation in a liquid medium wherein the concentration of gas generated by the fermenting liquid is controlled for at least part of the process by removing dissolved gas directly from the fermenting liquid by diffusion.

2. A process for reducing the level of foam generated during anaerobic fermentation in a liquid medium comprising controlling the concentration of generated gas in the fermenting liquid by removing dissolved gas therefrom by diffusion during at least part of the fermentation.

3. A process as claimed in Claim 1 or Claim 2, wherein said concentration of dissolved gas is below the saturation level.

4. A process as claimed in any one of the preceding claims, wherein the dissolved gas is removed from the liquid using a sheet of gas permeable membrane material.

5. A process as claimed in any one of Claims 1 to 3, wherein the dissolved gas is removed from the liquid using a hollow fibre of gas permeable membrane material.

6. A process as claimed in Claim 4 or Claim 5, wherein the dissolved gas is removed from the fermenting liquid using flow of nitrogen gas on the opposite side of the membrane to the fermenting liquid.

7. A process as claimed in Claim 4 or Claim 5, wherein the dissolved gas is removed from the fermenting liquid by applying a pressure differential using a vacuum on the opposite side of the membrane to the fermenting liquid or pressurising the liquid contact side of the membrane..

8. A process as claimed in any one of the preceding claims, wherein the gas removed by said diffusion is used in downstream processing of a fermentation product.

9. A process as claimed in any one of the preceding claims, wherein the gas is methane.

10. A process as claimed in any one of Claims 1 to 8, wherein the gas is CO₂.

11. A process as claimed in Claim 10, wherein the fermentation is the brewing of beer.

12. A process as claimed in any one of the preceding claims, wherein during startup of the fermentation process a molecular oxygen-containing gas is introduced into the fermenting liquid by diffusion.

13. The use of a gas permeable membrane in an anaerobic fermentation process to remove dissolved gas generated in a fermenting liquid to reduce the level of foam generated by controlling the concentration of gas in the liquid.

14. A use as claimed in Claim 13, wherein said concentration of dissolved gas is below the saturation level.

15. A use as claimed in Claim 12 or Claim 13, wherein the membrane is as defined in Claim 4 or 5.

16. A use as claimed in Claim 15, wherein the dissolved gas is removed as defined in Claim 6 or Claim 7.

17. A use as claimed in any one of Claims 13 to 16, wherein the gas is methane.

18. A use as claimed in any one of Claims 13 to 16, wherein the gas is carbon dioxide.

19. A use as claimed in Claim 18, wherein the fermentation is the brewing of beer.

20. An apparatus for carrying out a process as claimed in Claim 1 or Claim 2, said apparatus comprising a fermentation vessel 2 for anaerobic fermentation in a liquid medium, a separator unit 22; 66 comprising at least one gas permeable membrane 26 for the diffusion of gas from dissolution in the fermenting liquid, means 18, 20, 24, 28, 43, 44 for contacting the fermenting liquid with one side of the membrane 26; and means 36, 46 for removing gas diffusing through the membrane 26 to the other side thereof.

21. An apparatus as claimed in Claim 20, wherein the separator unit 66 is located in the fermentation vessel 2.

22. An apparatus as claimed in Claim 20 or Claim 21, wherein the separator unit 22 comprises at least one sheet of gas permeable membrane material.

23. An apparatus as claimed in Claim 20 or Claim 21, wherein the separator unit 22; 66 comprises at least one hollow fibre 26 of gas permeable membrane material.

24. An apparatus as claimed in any one of Claims 20 to 23 comprising means 50, 62; 68, 70 to apply a vacuum to said other side of the gas permeable membrane 26.

25. An apparatus as claimed in any one of Claims 20 to 23 comprising means 40, 38, 42, 34 to apply a flow of nitrogen gas to said other side of the gas permeable membrane.
